(19) Europäisches Patentamt
European Patent Office
Office européen des brevets

(11) **EP 4 512 321 B1**

(12) **EUROPEAN PATENT SPECIFICATION**

(45) Date of publication and mention
of the grant of the patent:
**13.05.2026 Bulletin 2026/20**

(21) Application number: **24181712.1**

(22) Date of filing: **12.06.2024**

(51) International Patent Classification (IPC):
*A61B 5/024* (2006.01)   *A61B 5/00* (2006.01)

(52) Cooperative Patent Classification (CPC):
**A61B 5/02416; A61B 5/681; A61B 5/7221;
A61B 5/7275; A61B 5/7282; A61B 5/746**

(54) **WEARABLE DEVICE FOR DETECTING A CARDIAC ARREST**

TRAGBARE VORRICHTUNG ZUR ERKENNUNG EINES HERZSTILLSTANDS

DISPOSITIF PORTABLE POUR DÉTECTER UN ARRÊT CARDIAQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **23.08.2023 NL 2035659**

(43) Date of publication of application:
**26.02.2025 Bulletin 2025/09**

(73) Proprietor: **Wear It System Holding S.A.
1228 Plan- les- Ouates (CH)**

(72) Inventors:
• **HANSE, Iddo Johanan Gideon
1228 Plan- les- Ouates (CH)**
• **EBRAHIMKHEIL, Kambiz
1228 Plan- les- Ouates (CH)**

(74) Representative: **van Breda, Jacobus
Octrooibureau Los & Stigter B.V.
P.O. Box 20052
1000 HB Amsterdam (NL)**

(56) References cited:
US-A1- 2017 209 055   US-A1- 2020 305 737
US-A1- 2022 249 026

• **BONOMI ALBERTO G ET AL: "Atrial fibrillation
detection using photo-plethysmography and
acceleration data at the wrist", 2016 COMPUTING
IN CARDIOLOGY CONFERENCE (CINC), CCAL,
11 September 2016 (2016-09-11), pages 277 - 280,
XP033071203**

**Description**

General description

The invention relates to a wearable device for non-invasively detecting a cardiac arrest of a wearer, using multi-wavelength photoplethysmography (PPG) signals obtained by said wearable device from peripheral blood vessels. This approach holds potential in enabling early detection of cardiac arrest, which is of paramount importance for improving patient outcomes and enhancing survival rates.

Photoplethysmography (PPG) is a non-invasive optical technique that measures blood volume changes in the microvascular bed of tissue. It works by shining a light source, typically an LED, onto the skin and detecting the amount of light that is transmitted or reflected back to a photodetector. This optical signal can be used to derive information about blood flow, heart rate, and other physiological parameters.

Scientific literature consistently underscores the criticality of timely recognition and intervention in cardiac arrest cases. Rapid identification of cardiac arrest triggers immediate initiation of life-saving measures, including vital procedures such as cardiopulmonary resuscitation (CPR) and defibrillation. Studies have unequivocally demonstrated that each minute of delay in commencing CPR and defibrillation reduces survival rates by 7-10%. Hence, early intervention significantly enhances the prospects of restoring normal heart rhythm, preventing irreversible damage to vital organs, and saving lives. Trained emergency response personnel and medical staff in hospitals or skilled nursing care facilities were the main users of defibrillator devices in the past, primarily when responding to a cardiac arrest incident at the location of the individual.

US20200305737 proposes a method for monitoring heart rhythm disturbance based PPG signals wherein a heart rhythm disturbance is detected when the PPG signals drop below a predetermined threshold.

Nowadays, there are widely available automated versions of defibrillators known as "automated external defibrillators" (AEDs). An automated external defibrillator (AED) is a portable electronic device used to treat sudden cardiac arrest (SCA). It is designed to deliver an electric shock to the heart, known as defibrillation, to restore a normal heartbeat. AEDs are user-friendly and equipped with built-in instructions to guide bystanders in their use. They analyze the heart's rhythm and determine if a shock is needed. When applied promptly, typically within minutes of SCA onset, AEDs greatly increase the chances of survival. These life-saving devices are often found in public spaces, workplaces, and healthcare settings to ensure immediate access during emergencies and are designed to be used by individuals with minimal or no medical training.

Promptly alerting individuals and emergency services when a cardiac arrest occurs remains a critical need. The presence of CPR training and automated AED technology, although widely available, may go unnoticed during the crucial initial minutes in various settings such as offices, homes, or care facilities. This oversight can lead to tragic outcomes, as potential responders may not be aware of the situation. Effective notification systems are vital to ensure swift intervention. By immediately notifying family members, neighbors, office workers, and care facility staff of a cardiac arrest incident, the chances of timely life-saving interventions significantly increase, and the risk of sudden cardiac death is reduced.

It is an object of the current invention to correct the shortcomings of the prior art and to provide a solution for accurate single-site measured PPG signals in order to detect potential cardiac arrests. This and other objects which will become apparent from the following disclosure, are provided with and a light-emitting wearable device, having the features of one or more of the appended claims.

In a first aspect of the invention, the light-emitting wearable device for processing a photoplethysmography signal and detecting a cardiac arrest of a wearer, wherein the photoplethysmography signal comprises a plurality of pulses wherein a pulse is the photoplethysmography signal between two consecutive valleys, wherein the wearable device is configured for:

- filtering the photoplethysmography signal by removing frequencies outside a predefined frequencies range using a bandpass filter;
- calculating at least one of the following values for each pulse of the filtered photoplethysmography signal:

  ○ a pulse wave amplitude left by calculating a difference between amplitudes of a first peak and a first valley of the pulse;
  ○ a pulse wave amplitude right by calculating a difference between amplitudes of the first peak and a second valley of the pulse;
  ○ a pulse wave duration by calculating a difference between a time of the second valley and a time of the first valley of the pulse;
  ○ a rise time by calculating a difference between a time of the first peak and a time of the first valley of the pulse;
  ○ a systolic-to-diastolic duration ratio by calculating the ratio between a difference between the rise time and a difference between the time of the second valley and the time of the first peak of the pulse;

- eliminating a photoplethysmography pulse when:

  ○ the rise time of said pulse is outside a first time range e.g. [0.01 - 10] seconds or [0.08 - 0.49] seconds; or
  ○ the pulse wave duration of said pulse is outside a second time range e.g. [0.01 -10] seconds or [0.3 - 2] seconds; or
  ○ a ratio between the pulse wave amplitude right and the pulse wave amplitude left is smaller than a first threshold value e.g. 5 or 3 or 0.4; or
  ○ the systolic-to-diastolic duration ration is larger a second threshold value e.g. 0.1 or 0.5 or 1.1;

- eliminating a current photoplethysmography pulse when:

  ○ a ratio between the rise time of a preceding photoplethysmography pulse and the rise time of said current photoplethysmography pulse is larger than a third threshold value e.g. 10% or 20% or 33%; or
  ○ a ratio between the pulse wave duration of a preceding photoplethysmography pulse and the pulse wave duration of said current photoplethysmography pulse is larger than a fourth threshold value e.g. 10% or 20% or 50%; or
  ○ a ratio between the pulse wave amplitude of a preceding photoplethysmography pulse and the pulse wave amplitude of said current photoplethysmography pulse is larger than a fifth threshold value e.g. 10% or 20% or 50%;

- calculating an average value of amplitudes of non-eliminated photoplethysmography pulses;
- monitoring a plurality of consecutive pulses (e.g. 4 pulses) after detecting a photoplethysmography pulse with a amplitude lower than a fraction (e.g. 10% or 20% or 33%) of said average value;
- triggering an alarm when an amplitude of said plurality of consecutive pulses is lower than said fraction of the average value.

A band-pass filter, such as second-order Butterworth band-pass filter, optionally with a frequency range of [0.2 - 10] Hz or [0.67 - 5] Hz can be applied to the PPG signals (preferably the green PPG signals) to filter out high-frequency noise and smooth the PPG pulse signals. This filtering will remove the large DC component (baseline signal) of the PPG data as well as some noises due to motion artifacts, ambient light and 50-60 Hz power line interference. What will remain after filtering is the AC component (PPG pulse signal) of the PPG.

Suitably, the wearable device of the current invention is configured for calculating an average value of amplitudes of non-eliminated photoplethysmography pulses. Said average value of amplitudes is the cardiac arrest threshold value. Once the amplitude of the peak of the PPG signal is below fraction (e.g. 33%) of the average value, a potential cardiac arrest event is detected.

After determining the threshold value for which the PPG amplitude is seen as potential cardiac arrest, it is preferable to continue monitoring the quality of the PPG signal to determine if there is still a normal PPG signal available, but with a small amplitude, or if the PPG signal is no longer detected at all. To confirm the potential cardiac arrest event the method of the current invention comprises the steps of monitoring a plurality of consecutive pulses after detecting a photoplethysmography pulse with a amplitude lower than a fraction of said average value, and triggering an alarm when an amplitude of said plurality of consecutive pulses is lower than said fraction of the average value. If a normal PPG pulse with low amplitude is detected, no alarm is given. Once a plurality (e.g. four) consecutive normal PPG pulses above the threshold are detected it is preferrable to go back to the step of monitoring the quality of the PPG signal. If below the threshold no normal PPG pulse is detected, there still is a potential cardiac arrest.

It is preferable to only clear the potential cardiac arrest when a plurality (e.g. four) of consecutive normal pulses are detected within a first time duration (e.g. 3 seconds). The plurality of consecutive normal pulses are defined by a PPG pulse when the signal quality index is indicates a normal pulse. For instance, when for the first time duration (e.g. 3 seconds) no PPG signal is detected but then there is one normal PPG pulse and after that the PPG signal is gone again, the risk for a potential cardiac arrest will not be cleared.

Advantageously, the wearable device is configured for stopping the alarm when detecting, within the first time duration (e.g. 5 seconds), a plurality of consecutive pulses (e.g. four) having amplitudes higher than said fraction of the average value of amplitudes of non-eliminated photoplethysmography pulses.

More advantageously, the wearable device is configured for stopping the alarm when:

- the wearer presses a button on the wearable device for indicating a false alarm; and
- the wearer presses said button within a preset second time duration (e.g. 10 seconds).

It is convenient to alert close ones, emergency services and other third parties of the cardiac arrest event. For that end, the method conveniently comprises the steps of using GPS components of the wearable device to collect location coordinates of the wearer and sharing said location coordinates with a third party.

To save the battery life and the processing power of the wearable device, the wearable device is configured for performing the steps of the method according to the preceding operations only when a wearing detection test is positive wherein said test comprises the steps of:

- registering a lowest amplitude and a highest amplitude of the filtered photoplethysmography signal during a third time duration (e.g. 6 seconds) after detecting a pulse wave with a amplitude higher than a sixth threshold value (e.g. 2x highest amplitude of the PPG within the third time duration);
- calculating a fluctuation range by subtracting the lowest amplitude from the highest amplitude; and
- registering a negative result to the wearing detection test when the fluctuation range is lower than a seventh threshold value.

When the wearable device is worn correctly on the wrist of the wearer, the PPG data includes small fluctuations caused by physiological signals like heart rate and blood flow. The moment someone takes off the bracelet from the wrist, a big and short jump is overserved in the PPG data due to big movements of the bracelet in the air. It is, therefore, preferable to first checks for any big jumps in the PPG data. If no jump is detected, the state of the wearing will stay the same. If a jump on the PPG data is detected, the method comprises checking for the fluctuations in the PPG signal.

Furthermore, the wearable device is configured for:

- calculating a mean value of the lowest amplitudes of a plurality of wearers wearing the wearable device for a plurality of hours; and
- setting the seventh threshold value at a fraction (e.g. 20%) of said mean value.

Advantageously, the wearable device is configured for performing a wearing detection test comprising the steps of:

- measuring an acceleration of the wearable device movements;
- registering a negative result to the wearing detection test when said acceleration is lower than an eighth threshold value during a fourth time duration (e.g. 6 seconds).

More advantageously, the wearable device is configured for:

- measuring the acceleration of the wearable device movements and collecting a lowest acceleration of the wearable device movements during a fifth time duration (e.g. 5 seconds);
- calculating a mean value of the lowest acceleration of the wearable device movements of a plurality of wearers; and
- setting the eighth threshold value at a fraction (e.g. 20%) of said mean value.

Suitably, the wearable device is configured for of measuring an acceleration of the wearable device movements comprising the steps of:

- collecting acceleration data of axis x, axis y and axis z from an accelerometer imbedded in the wearable device;
- calculating a time derivative of the acceleration data of axis x, a time derivative of the acceleration data of axis y and a time derivative of the acceleration data of axis z; and
- calculating the acceleration of the wearable device movement by calculating a square root of a sum of each time derivative of the acceleration data squared.

Additionally, the wearable device of the current invention is configured for registering a fall event when the acceleration of the wearable device movement is larger than a nineth threshold value (e.g. 3g, wherein g=9.8m/s) within a sixth time duration (e.g. 2500ms).

Advantageously, the wearable device of the current invention is configured for:

- collecting a timestamp of peak acceleration when registering the fall of the wearer within the sixth time duration;
- collecting a timestamp of impact start when the acceleration of the wearable device movement is lower than a tenth threshold value (e.g. 0.8g) within the sixth time duration (e.g. 2500 ms);
- collecting a timestamp of impact end when the acceleration of the wearable device movement is larger than an eleventh threshold value (e.g. 1.5g) within the sixth time duration; and
- calculating acceleration parameters proportional to the acceleration of the wearable device movement at each time stamp or at an arithmetic combination of at least two timestamps.

More advantageously, the wearable device of the current invention is configured for training a neural network using a gradient boosting model and a training dataset comprising the calculated acceleration parameters and the registered fall events by minimizing a loss function on the training dataset.

In a second embodiment of the invention, the wearable device comprises a computer loaded with a computer program wherein said program is arranged for causing the computer to carry out the steps of the operations according to any one of aforementioned steps.

The invention will hereinafter be further elucidated with reference to the drawing of an exemplary embodiment of a light-emitting wearable device according to the invention that is not limiting as to the appended claims.

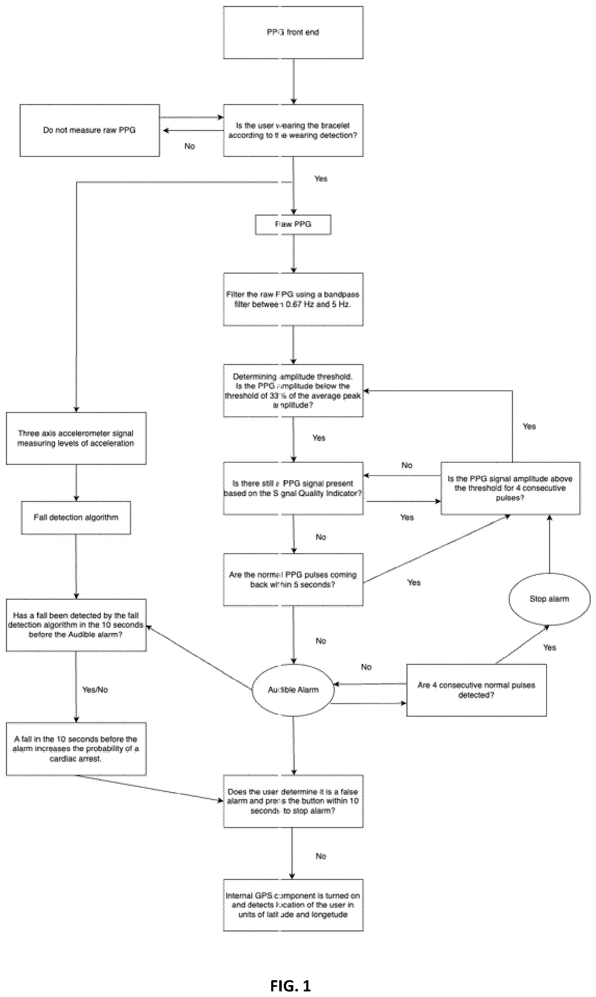

In the drawing, figure 1 shows a schematic diagram for the actions performed by the wearable device according to the invention.

Whenever in the figures the same reference numerals are applied, these numerals refer to the same parts.

Detailed description

*The PPG front end:*

The PPG front end part of the method is designed to detect and measure optical signals emitted by the LED lights which is reflected back from the skin of the wearer. The LEDs under the wearable device emit light (green, red and infrared) into the user's skin, and the photodiodes capture the amount of light that is reflected back. This reflected light contains valuable information about the wearer's blood perfusion, which is the variation in blood volume in the wrist's blood vessels.

The PPG front end comprises using an analog front end (AFE) circuit to cancel out any interference caused by ambient light. Since ambient light can significantly impact the accuracy of PPG readings, the AFE helps minimizing its influence, ensuring more reliable results. Then the AFE converts the analog PPG signals into a digital format, making them easier to process and analyze. These digital PPG signals contain oscillations caused by changes in blood perfusion, providing valuable insights into the wearer's pulse rate, respiration rate, blood oxygen levels, blood pressure, etc.

The PPG front end comprises using a configurable LED driver, which allows the microcontroller of the wearable device to control the power output to the LEDs. This control enables adjustments in the intensity and timing of the LED pulses, optimizing the PPG measurements and enhancing the overall performance of the sensor system to cope with different skin colors and different wearing conditions. Every second the PPG value of every channel (i.e. color; green, red, infrared) is processed (independently from other channels). If the mean PPG value is less than an expected threshold, then the microcontroller will increase the emitting power of the corresponding LED. If the PPG value is more than a certain threshold the LED power will be decreased.

The microcontroller of the wearable device can also control the sampling frequency of the PPG signals from 25 Hz to 256 Hz depending on the requirements of the algorithms.

*Wearing detection:*

The wearing detection step of the invention comprises the step of utilizing the PPG (Photoplethysmography) and/or accelerometer (Acc) data to determine whether the wearable device is being worn by the user. Here's how it works:

- PPG Data: When the wearable device is worn correctly on the wrist, the PPG data includes fluctuations caused by physiological signals like heart rate and blood flow. If the bracelet is not being worn, the PPG sensor will not receive the expected signals, and the PPG data will not show normal patterns of the pulse rate.
- Accelerometer Data: The wearable device comprises an accelerometer, which is used to detects motion and orientation changes. The accelerometer can measure the acceleration forces acting on the bracelet. When a user wears the wearable device, their wrist movements and the gravitational forces acting on the device generate distinct patterns of acceleration.
- Combined Analysis and algorithm: the PPG and accelerometer data can be simultaneously analyzed. This analysis determines whether the bracelet is being worn. Wearing detection algorithms use predefined thresholds and machine learning techniques to classify the combined PPG and accelerometer data. These algorithms are trained on large datasets to accurately identify the patterns associated with wearing and non-wearing scenarios.

*Band pass filtering:*

A second-order Butterworth band-pass filter is applied to the green PPG signals to filter out high-frequency noise and smooth the PPG pulse signals. This filtering will remove the large DC component (baseline signal) of the PPG data as well as some noises due to motion artifacts, ambient light and 50-60 Hz power line interference. What will remain after filtering is the AC component (PPG pulse signal) of the PPG. This filtering process is applied to the PPG green continuously.

*Calculating threshold values:*

The high and low peaks of filtered PPG signals are determined as the local maxima/minima points by a comparison of neighboring values. Suitably, the of the wearable device of the current invention is configured for extracting four features are for every "pulse" of PPG signal. A "pulse" is defined as one low peak (AKA valley) to the next low peak.

To assess the quality of the PPG signals, the the wearable device of the current invention is configured for calculating the following values:

Pulse wave amplitude left: $PWA_{left} = V_{peak1} - V_{valley1}$.
Pulse wave amplitude right: $PWA_{right} = V_{peak1} - V_{valley2}$.
Pulse wave duration: $PWD = T_{valley2} - T_{valley1}$
Rise time: $RT = T_{peak1} - T_{valley1}$

Systolic-to-diastolic duration ratio:

$$SDR = (T_{peak1} - T_{valley1}) / (T_{valley1} - T_{peak1}).$$

Advantageously, the wearable device of the current invention comprises the step of eliminating the low quality ppg signals when at least one of the conditions below holds:

- The RT is outside a first time range such as [0.08 - 0.49] seconds;
- The PWD is outside a second time range such as [0.3 - 2] seconds;
- The $\min\{PWA_{right}/PWA_{left}, PWA_{left}/PWA_{right}\}$ is smaller than a first threshold value such as 0.4.
- The SDR is larger than a second threshold value such as 1.1.

In the next stage, the feature varieties of the neighbor pulses are considered. There are three conditions. If the pulse fits any condition, the quality of the pulse is annotated low and is eliminated. The three conditions are explained as following (n represents the nth pulse):

- The ratio of RT(n-1) and RT(n) is larger than a third threshold value such as 33%.
- The ratio of PWD(n-1) and PWD(n) is larger than a fourth threshold value such as 50%.
- The ratio of PWA(n-1) and PWA(n) is larger than a fifth threshold value such as 50%.

By eliminating the low quality PPG pulses, only PPG signals with normal quality are used to determine the cardiac arrest threshold for which the start of a potential cardiac arrest is detected.

After determining the threshold value for which the PPG amplitude is seen as potential cardiac arrest, the wearable device of the current invention is configured for determining whether there is still a ppg signal available, but with a small amplitude, or whether the PPG signal is no longer detected at all.

The potential cardiac arrest is only clearer when a plurality of consecutive normal pulses (e.g. four) are detected. For instance, when for the first time duration (e.g. 3 seconds) no PPG signal is detected but then there is one normal PPG pulse and after that the PPG signal is gone again, the risk for a potential cardiac arrest will not be cleared.

If the PPG signal is no longer detected within the first time duration, an alarm is triggered. The alarm is stopped only if a plurality (e.g. four) consecutive normal PPG pulses are detected.

*Fall detection:*

The fall detection step is used as an extra check to increase the probability of the cardiac arrest. The wearer may be on their feet when the cardiac arrest occurs. Normally this leads to the user than falling as there is no cardiac output.

An accelerometer is a device that measures proper acceleration. Proper acceleration is the rate of change in velocity of a body in its own rest state. Proper acceleration contains changes in velocity for X,Y and Z axis. Accelerometer data (32 Hz) comprise data for X, Y and Z axis.

The data can be processed in 2500ms intervals (windows). For every window the total acceleration is calculated with the formula below, where t denotes a timestep in a window. total acceleration $(\mathtt{t}) = \sqrt{(Xt^2 + Yt^2 + Zt^2)}$

The wearable device includes an accelerometer, which can be used to detects motion and orientation changes. The accelerometer can measure the acceleration forces acting on the wearable device. When a user wears it, their wrist movements and the gravitational forces acting on the device generate distinct patterns of acceleration. To detect movements intensity, the difference acceleration vector in each axis is calculated:

```
d_acc_x = acc_x(t) - acc_x(t-1)

d_acc_y = acc_y(t) - acc_y(t-1)

d_acc_z = acc_z(t) - acc_z(t-1)
```

where acc_x(t), acc_y(t), acc_z(t) denote respectively the accelerometer data of axis x, y and z at time "t", and then the norm of the vector would be an indication of the movement intensity: N_acc = SQRT [(d_acc_x)$^2$ + (d_acc_y)$^2$ + (d_acc_z)$^2$].

If N_acc is smaller than a threshold T_acc for 6 consecutive seconds, "no movement" (NMOV) is declared. The threshold T_acc is defined as 20% of the "average low N_acc" (ALN_acc). The ALN_acc is estimated experimentally by calculating the mean of the minimum N_acc of a population of users wearing the device day and night for 24 hours.

- Combined Analysis and algorithm: The two above processes are combined and done simultaneously in one process. This process determines if the bracelet is being worn or not. If NPF and NMOV are detected at the same time, "not wearing" is declared.

*Window shifting:*

After the data is processed windows of 2500ms can be created.
For every window the highest peak of total acceleration is selected only if there are no other peaks in the window [t-2500ms, t]. Following that, the selected peak can be marked as peak time (pt) if the acceleration for that timestep (a_t) is larger than 3g. This shows a peak in total acceleration has been found, which could entail a possible fall event. If this criterium is not met, the window is discarded and shifts forward.
To verify the peak, two timestamps are collected, impact end (*ie*) and impact start (*is*). These features denote the last time the total acceleration was above the eleventh threshold value (e.g. 1.5 g), and the first time the total acceleration was below a tenth threshold value (e.g. 0.8g) respectively. In addition, (*ie*) should be within [t-2500ms, t] and (*is*) should be within [ie-1200ms, pt]. If (*ie*) is not found, then it is fixed to (*ie*) = *pt* + 1000ms.
In the peak detection step of the method, three features are obtained: pt, *ie* and *is*. These features are used to calculate the acceleration parameters that are proportional to the acceleration of the wearable device movement at each time stamp or at an arithmetic combination of at least two timestamps:
Average Absolute Acceleration Magnitude Variation, where N denotes the number of timesteps in the window:

$$AAMV = \sum_{t=is}^{ie} \frac{|a_{t+1} - a_t|}{N}$$

Maximum Peak Index:

$$MPI = max_{t \in [is,ie]} (a_t)$$

Minimum Valley Index

$$MVI = min_{t \in [is-500, ie]} (a_t)$$

Peak Duration Index, with ps being the start of the detected peak, defined by the last timestep where the total acceleration was lower than 1.8 g before pt. pe is denoted as the end of a peak and is the first time where the total acceleration is lower than 1.8 g after pt.

$$PDI = pe - ps$$

Activity Ratio Index, ARI, calculated as the ratio between the number of samples not in [0.85 g, 1.3g] and the total number of timesteps in the 700-ms interval centered around (is + ie) / 2.

Free Fall Index, FFI, the average magnitude in the interval [t_FFI,pt], where tFFi is denoted as the time between the first total acceleration below 0.8 g occurring up to 200 ms before pt, if not found we set this value to [pt - 2200, pt].

Step Count Index, SCI, measured as the number of peaks in the interval [pt - 2200 ms, pt].

Using these features, time windows can be flagged as possible fall events and stored as an event to train a prediction model in the next step of the method of the current invention.

*Model and inference:*

To train the fall detection model the wearable device of the current invention is configured for using a dataset $W = \{w_0, w_1, ..., w_t\}$, where $w_i$ denotes a data sample (window) that consists of features $x_i \in X$ and a label $y_i \in Y$, with $i, t \in \mathbb{N}$. $y_i$ yields 1 if a window is a fall event and 0 if a window is a false fall event.

| W | AAMV | IDI | MPI | MVI | PDI | ARI | FFI | SCI | Fall or no Fall |
|---|---|---|---|---|---|---|---|---|---|
| w0 | ±2.753 | 0.215 | 105 | 105 | 0.07 | ±0.314 | 1.06 | 1 | 0 |
| w1 | ±4.512 | 0.72 | 40 | 101 | 0.205 | ±0.907 | ±2.012 | 1 | 0 |
| w2 | ±1.900 | 0.54 | 99 | 179 | 0.075 | ±0.184 | ±1.009 | 0 | 1 |

Let W be w0, w0 is denoted as an event consisting of :

$$x0 = \{2.753, 0.215, 105, 0.07, 0.314, 1.06, 1\}$$

$$y0 = 0$$

With the obtained dataset a cis trained and hypertuned. The Gradient Boosting Model combines the predictions of several weak learners to adjust the model weights of the overall model. Since, the practical application of this model should have low false positives and a high accuracy the training of the neural network optimizes for those tasks.

After the model has been trained, inference can be done on new data samples. The accuracy of the model will only increase until convergence by the *Law of Large Numbers*. Therefore, the model will be continuously optimized and trained with new data points obtained after deployment.

Although the invention has been discussed in the foregoing with reference to an exemplary embodiment of the device of the invention, the invention is not restricted to this particular embodiment which can be varied in many ways without departing from the invention. The discussed exemplary embodiment shall therefore not be used to construe the append-ed claims strictly in accordance therewith. On the contrary the embodiment is merely intended to explain the wording of the appended claims without intent to limit the claims to this exemplary embodiment. The scope of protection of the invention shall therefore be construed in accordance with the appended claims only, wherein a possible ambiguity in the wording of the claims shall be resolved using this exemplary embodiment.

Variations and modifications of the present invention will be obvious to those skilled in the art

Optionally, embodiments of the present invention can include a general or specific purpose computer or distributed system programmed with computer software implementing steps described above, which computer software may be in any appropriate computer language, including but not limited to C++, FORTRAN, ALGOL, BASIC, Java, Python, Linux, assembly language, microcode, distributed programming languages, etc. The apparatus may also include a plurality of such computers / distributed systems (e.g., connected over the Internet and/or one or more intranets) in a variety of hardware implementations. For example, data processing can be performed by an appropriately programmed microprocessor, computing cloud, Application Specific Integrated Circuit (ASIC), Field Programmable Gate Array (FPGA), or the like, in conjunction with appropriate memory, network, and bus elements. One or more processors and/or microcontrollers can operate via instructions of the computer code and the software is preferably stored on one or more tangible non-transitive memory-storage devices.

**Claims**

1. A light-emitting wearable device for processing a photoplethysmography signal and detecting a cardiac arrest of a wearer, wherein the photoplethysmography signal comprises a plurality of pulses wherein a pulse is the photoplethysmography signal between two consecutive valleys, **characterized in that** the wearable device is configured for:

- calculating at least one of the following values for a pulse of the photoplethysmography signal:

  ◦ a pulse wave amplitude left by calculating a difference between amplitudes of a first peak and a first valley of the pulse;
  ◦ a pulse wave amplitude right by calculating a difference between amplitudes of the first peak and a second valley of the pulse;
  ◦ a pulse wave duration by calculating a difference between a time of the second valley and a time of the first valley of the pulse;
  ◦ a rise time by calculating a difference between a time of the first peak and a time of the first valley of the pulse;
  ◦ a systolic-to-diastolic duration ratio by calculating the ratio between the rise time and a difference between the time of the second valley and the time of the first peak of the pulse;

- eliminating a photoplethysmography pulse when:

  ◦ the rise time of said pulse is outside a first time range; or
  ◦ the pulse wave duration of said pulse is outside a second time range; or
  ◦ a ratio between the pulse wave amplitude right and the pulse wave amplitude left is smaller than a first threshold value; or
  ◦ the systolic-to-diastolic duration ratio is larger a second threshold value;

- eliminating a current photoplethysmography pulse when:

  ◦ a ratio between the rise time of a preceding photoplethysmography pulse and the rise time of said current photoplethysmography pulse is larger than a third threshold value; or
  ◦ a ratio between the pulse wave duration of a preceding photoplethysmography pulse and the pulse wave duration of said current photoplethysmography pulse is larger than a fourth threshold value; or
  ◦ a ratio between the pulse wave amplitude of a preceding photoplethysmography pulse and the pulse wave amplitude of said current photoplethysmography pulse is larger than a fifth threshold value;

- calculating an average value of amplitudes of non-eliminated photoplethysmography pulses;
- monitoring a predetermined number of plural consecutive pulses after detecting a photoplethysmography pulse with an amplitude lower than a pre-determined fraction of said average value;
- triggering an alarm when an amplitude of said plurality of consecutive pulses is lower than said fraction of the average value.

2. The wearable device according to claim 1, **characterized in that** the wearable device is configured for filtering the photoplethysmography signal by preferably removing frequencies outside a predefined frequencies range using a bandpass filter, and using said filtered photoplethysmography signal in the remaining steps of the method.

3. The wearable device according to claim 1 or 2, **characterized in that** the wearable device is configured for stopping the alarm when detecting, within a preset first time duration, a plurality of consecutive pulses having amplitudes higher than said fraction of the average value.

4. The wearable device according to any one of claims 1 - 3, **characterized in that** the wearable device is configured for stopping the alarm when:

- the wearer presses a button on the wearable device for indicating a false alarm; and
- the wearer presses said button within a preset second time duration.

5. The wearable device according to any one of claims 1 - 4, **characterized in that** wearable device is configured for using GPS components of the wearable device to collect location coordinates of the wearer and sharing said location coordinates with a third party.

6. The wearable device according to any one of claims 1 - 5, **characterized in that** the wearable device is configured for performing the operations of claims 1-5 only when a result of a wearing detection test is positive.

7. The wearable device according to claim 6, **characterized in that** the wearable device is configured for:

- registering a lowest amplitude and a highest amplitude of the filtered photoplethysmography signal during a third time duration after detecting a pulse wave with an amplitude higher than a sixth threshold value;
- calculating a fluctuation range by subtracting the lowest amplitude from the highest amplitude; and
- registering a negative result to the wearing detection test when the fluctuation range is lower than a seventh threshold value, and registering a positive result to the wearing detection test when the fluctuation range is higher than the seventh threshold value.

8. The wearable device according to claim 7, **characterized in that** the wearable device is configured for:

- calculating a mean value of the lowest amplitudes of a plurality of wearers; and
- setting the seventh threshold value at a fraction of said mean value.

9. The wearable device according to any one of claims 6 - 8, **characterized in that** the wearing detection test comprises the steps of:

- measuring an acceleration of the wearable device movements; and
- registering a negative result to the wearing detection test when said acceleration is lower than an eighth threshold value during a fourth time duration, registering a positive result to the wearing detection test when said acceleration is higher than the eighth threshold value during the fourth time duration.

10. The wearable device according to claim 9, **characterized in that** wearable device is configured for:

- measuring the acceleration of the wearable device movements and collecting a lowest acceleration of the wearable device movements during a fifth time duration;
- calculating a mean value of the lowest acceleration of the wearable device movements of a plurality of wearers; and
- setting the eighth threshold value at a fraction of said mean value.

11. The wearable device according to claim 10, **characterized in that** wearable device is configured for measuring an acceleration of the wearable device movements comprises the steps of:

- collecting acceleration data of axis x, axis y and axis z from an accelerometer imbedded in the wearable device;
- calculating a time derivative of the acceleration data of axis x, a time derivative of the acceleration data of axis y and a time derivative of the acceleration data of axis z;
- calculating the acceleration of the wearable device movement by calculating a square root of a sum of each time derivative of the acceleration data squared.

12. The wearable device according to any one of claims 1 - 11, **characterized in that** wearable device is configured for registering a fall event when the acceleration of the wearable device movement is larger than a ninth threshold value within a sixth time duration.

13. The wearable device according to claim 12, **characterized in that** the wearable device is configured for:

- collecting a timestamp of peak acceleration when registering the fall of the wearer within the sixth time duration;
- collecting a timestamp of impact start when the acceleration of the wearable device movement is lower than a tenth threshold value within the sixth time duration;

- collecting a timestamp of impact end when the acceleration of the wearable device movement is larger than an eleventh threshold value within the sixth time duration; and
- calculating acceleration parameters proportional to the acceleration of the wearable device movement at each time stamp or at an arithmetic combination of at least two timestamps.

14. The wearable device according to any one of claims 12 - 13, **characterized in that** the wearable device is configured for training a neural network for detecting a cardiac arrest using a gradient boosting model and a training dataset comprising the calculated acceleration parameters and the registered fall events by minimizing a loss function on the training dataset.

15. The wearable device according to any one of the preceding claims wherein said wearable device comprises a computer loaded with a computer program, **characterized in that** said program is arranged for causing the computer to carry out the operations according to any one of preceding claims.

**Patentansprüche**

1. Lichtemittierende tragbare Vorrichtung zum Verarbeiten eines Photoplethysmographie-Signals und zum Erkennen eines Herz-Kreislauf-Stillstands eines Trägers, wobei das Photoplethysmographie-Signal eine Vielzahl von Pulsen aufweist, wobei ein Puls das Photoplethysmographie-Signal zwischen zwei aufeinanderfolgenden Tälern ist, **dadurch gekennzeichnet, dass** die tragbare Vorrichtung dazu ausgebildet ist:

- zumindest einen der folgenden Werte für einen Puls des Photoplethysmographie-Signals zu berechnen:

  ∘ eine Pulswellenamplitude links durch Berechnen einer Differenz zwischen Amplituden einer ersten Spitze und eines ersten Tals des Pulses;
  ∘ eine Pulswellenamplitude rechts durch Berechnen einer Differenz zwischen Amplituden der ersten Spitze und eines zweiten Tals des Pulses;
  ∘ eine Pulswellendauer durch Berechnen einer Differenz zwischen einer Zeit des zweiten Tals und einer Zeit des ersten Tals des Pulses;
  ∘ eine Anstiegszeit durch Berechnen einer Differenz zwischen einer Zeit der ersten Spitze und einer Zeit des ersten Tals des Pulses;
  ∘ ein Verhältnis von systolischer zu diastolischer Dauer durch Berechnen des Verhältnisses zwischen der Anstiegszeit und einer Differenz zwischen der Zeit des zweiten Tals und der Zeit der ersten Spitze des Pulses;

- einen Photoplethysmographie-Puls zu eliminieren, wenn:

  ∘ die Anstiegszeit des Pulses außerhalb eines ersten Zeitbereichs liegt; oder
  ∘ die Pulswellendauer des Pulses außerhalb eines zweiten Zeitbereichs liegt; oder
  ∘ ein Verhältnis zwischen der Pulswellenamplitude rechts und der Pulswellenamplitude links kleiner als ein erster Schwellenwert ist; oder
  ∘ ein Verhältnis von systolischer zu diastolischer Dauer größer als ein zweiter Schwellenwert ist;

- einen aktuellen Photoplethysmographie-Puls zu eliminieren, wenn:

  ∘ ein Verhältnis zwischen der Anstiegszeit eines vorhergehenden Photoplethysmographie-Pulses und der Anstiegszeit des aktuellen Photoplethysmographie-Pulses größer als ein dritter Schwellenwert ist; oder
  ∘ ein Verhältnis zwischen der Pulswellendauer eines vorangegangenen Photoplethysmographie-Pulses und der Pulswellendauer des aktuellen Photoplethysmographie-Pulses größer als ein vierter Schwellenwert ist; oder
  ∘ ein Verhältnis zwischen der Pulswellen-Amplitude eines vorangegangenen Photoplethysmographie-Pulses und der Pulswellen-Amplitude des aktuellen Photoplethysmographie-Pulses größer als ein fünfter Schwellenwert ist;

- einen Durchschnittswert von Amplituden nicht eliminierter Photoplethysmographie-Pulse zu berechnen;
- eine vorbestimmte Anzahl einer Vielzahl aufeinanderfolgender Pulse nach dem Erkennen eines Photoplethysmographie-Pulses mit einer Amplitude, die kleiner als ein vorbestimmter Bruchteil des Durchschnittswerts ist, zu überwachen;
- einen Alarm auszulösen, wenn eine Amplitude der Vielzahl aufeinanderfolgender Pulse kleiner als der Bruchteil des Durchschnittswerts ist.

2. Tragbare Vorrichtung gemäß Anspruch 1, **dadurch gekennzeichnet, dass** die tragbare Vorrichtung dazu ausgebildet ist, das Photoplethysmographie-Signal zu filtern, indem sie vorzugsweise Frequenzen außerhalb eines vordefinierten Frequenzbereichs unter Verwendung eines Bandpassfilters entfernt, und das gefilterte Photoplethysmographie-Signal in den verbleibenden Schritten des Verfahrens verwendet.

3. Tragbare Vorrichtung gemäß Anspruch 1 oder 2, **dadurch gekennzeichnet, dass** die tragbare Vorrichtung dazu ausgebildet ist, den Alarm zu stoppen, wenn sie innerhalb einer voreingestellten ersten Zeitdauer eine Vielzahl aufeinanderfolgender Pulse mit Amplituden, die höher als der Bruchteil des Durchschnittswerts sind, erkennt.

4. Tragbare Vorrichtung gemäß einem der Ansprüche 1 - 3, **dadurch gekennzeichnet, dass** die tragbare Vorrichtung dazu ausgebildet ist, den Alarm zu stoppen, wenn:

   - der Träger eine Taste an der tragbaren Vorrichtung drückt, um einen Fehlalarm anzuzeigen; und
   - der Träger die genannte Taste innerhalb einer voreingestellten zweiten Zeitdauer drückt.

5. Tragbare Vorrichtung gemäß einem der Ansprüche 1 - 4, **dadurch gekennzeichnet, dass** die tragbare Vorrichtung dazu ausgebildet ist, GPS-Komponenten der tragbaren Vorrichtung zu verwenden, um Standortkoordinaten des Trägers zu sammeln und die Standortkoordinaten an einen Dritten weiterzugeben.

6. Tragbare Vorrichtung gemäß einem der Ansprüche 1 - 5, **dadurch gekennzeichnet, dass** die tragbare Vorrichtung dazu ausgebildet ist, die Vorgänge der Ansprüche 1 - 5 nur dann auszuführen, wenn ein Ergebnis eines Trageerkennungstests positiv ist.

7. Tragbare Vorrichtung gemäß Anspruch 6, **dadurch gekennzeichnet, dass** die tragbare Vorrichtung dazu ausgebildet ist:

   - eine niedrigste Amplitude und eine höchste Amplitude des gefilterten Photoplethysmographie-Signals während einer dritten Zeitdauer nach dem Erkennen einer Pulswelle mit einer Amplitude, die höher als ein sechster Schwellenwert ist, zu registrieren;
   - einen Schwankungsbereich durch Subtrahieren der niedrigsten Amplitude von der höchsten Amplitude zu berechnen; und
   - ein negatives Ergebnis für den Trageerkennungstest zu registrieren, wenn der Schwankungsbereich unter einem siebten Schwellenwert liegt, und ein positives Ergebnis für den Trageerkennungstest zu registrieren, wenn der Schwankungsbereich größer als der siebte Schwellenwert.

8. Tragbare Vorrichtung gemäß Anspruch 7, **dadurch gekennzeichnet, dass** die tragbare Vorrichtung dazu ausgebildet ist:

   - einen Mittelwert der niedrigsten Amplituden mehrerer Träger zu berechnen; und
   - den siebten Schwellenwert auf einen Bruchteil des Mittelwerts festzulegen.

9. Tragbare Vorrichtung gemäß einem der Ansprüche 6 - 8, **dadurch gekennzeichnet, dass** der Trageerkennungstest die folgenden Schritte aufweist:

   - Messen einer Beschleunigung der Bewegungen der tragbaren Vorrichtung; und
   - Registrieren eines negativen Ergebnisses für den Trageerkennungstest, wenn die Beschleunigung während einer vierten Zeitdauer niedriger als ein achter Schwellenwert ist, Registrieren eines positiven Ergebnisses für den Trageerkennungstest, wenn die Beschleunigung während der vierten Zeitdauer höher als der achte Schwellenwert ist.

10. Tragbare Vorrichtung gemäß Anspruch 9, **dadurch gekennzeichnet, dass** die tragbare Vorrichtung dazu ausgebildet ist:

   - die Beschleunigung der Bewegungen der tragbaren Vorrichtung zu messen und eine niedrigste Beschleunigung der Bewegungen der tragbaren Vorrichtung während einer fünften Zeitdauer zu erfassen;
- einen Mittelwert der niedrigsten Beschleunigung der Bewegungen der tragbaren Vorrichtung einer Vielzahl von Trägern zu berechnen; und
- den achten Schwellenwert auf einen Bruchteil des Mittelwerts festzulegen.

**11.** Tragbare Vorrichtung gemäß Anspruch 10, **dadurch gekennzeichnet, dass** die tragbare Vorrichtung zum Messen einer Beschleunigung der Bewegungen der tragbaren Vorrichtung, das die folgenden Schritte aufweist:

- Sammeln von Beschleunigungsdaten der Achse x, der Achse y und der Achse z von einem in die tragbare Vorrichtung eingebetteten Beschleunigungsmesser;
- Berechnen einer zeitlichen Ableitung der Beschleunigungsdaten der Achse x, einer zeitlichen Ableitung der Beschleunigungsdaten der Achse y und einer zeitlichen Ableitung der Beschleunigungsdaten der Achse z;
- Berechnen der Beschleunigung der Bewegung der tragbaren Vorrichtung durch Berechnen einer Quadratwurzel aus einer Summe der Quadrate jeder zeitlichen Ableitung der Beschleunigungsdaten ausgebildet ist.

**12.** Tragbare Vorrichtung gemäß einem der Ansprüche 1 - 11, **dadurch gekennzeichnet, dass** die tragbare Vorrichtung dazu ausgebildet ist, ein Sturzereignis zu registrieren, wenn die Beschleunigung der Bewegung der tragbaren Vorrichtung innerhalb einer sechsten Zeitdauer größer als ein neunter Schwellenwert ist.

**13.** Tragbare Vorrichtung gemäß Anspruch 12, **dadurch gekennzeichnet, dass** die tragbare Vorrichtung dazu ausgebildet ist:

- einen Zeitstempel der Spitzenbeschleunigung zu sammeln, wenn der Sturz des Trägers innerhalb der sechsten Zeitdauer registriert wird;
- einen Zeitstempel des Beginns des Aufpralls zu sammeln, wenn die Beschleunigung der Bewegung der tragbaren Vorrichtung innerhalb der sechsten Zeitdauer niedriger als ein zehnter Schwellenwert ist;
- einen Zeitstempel des Endes des Aufpralls zu sammeln, wenn die Beschleunigung der Bewegung der tragbaren Vorrichtung innerhalb der sechsten Zeitdauer größer als ein elfter Schwellenwert ist; und
- Beschleunigungsparameter, die proportional zu der Beschleunigung der Bewegung der tragbaren Vorrichtung zu jedem Zeitstempel oder zu einer arithmetischen Kombination von zumindest zwei Zeitstempeln sind, zu berechnen.

**14.** Tragbare Vorrichtung gemäß einem der Ansprüche 12 - 13, **dadurch gekennzeichnet, dass** die tragbare Vorrichtung dazu ausgebildet ist, ein neuronales Netzwerk zum Erkennen eines Herz-Kreislauf-Stillstands unter Verwendung eines Gradient-Boosting-Modells und eines Trainingsdatensatzes, der die berechneten Beschleunigungsparameter und die registrierten Sturzereignisse aufweist, zu trainieren, indem eine Verlustfunktion auf dem Trainingsdatensatz minimiert wird.

**15.** Tragbare Vorrichtung gemäß einem der vorstehenden Ansprüche, wobei die tragbare Vorrichtung einen Computer aufweist, auf dem ein Computerprogramm geladen ist, **dadurch gekennzeichnet, dass** das Programm dazu ausgelegt ist, den Computer zu veranlassen, die Operationen gemäß einem der vorstehenden Ansprüche auszuführen.

**Revendications**

**1.** Dispositif portable émettant de la lumière destiné au traitement d'un signal de photopléthysmographie et à la détection d'un arrêt cardiaque d'un porteur, le signal de photopléthysmographie comprenant une pluralité d'impulsions, une impulsion étant définie comme le signal de photopléthysmographie compris entre deux vallées consécutives, **caractérisé en ce que** le dispositif portable est configuré pour:

- calculer au moins l'une des valeurs suivantes pour une impulsion du signal de photopléthysmographie:

  ◦ une amplitude d'onde de pouls gauche en calculant une différence entre les amplitudes d'un premier pic et d'une première vallée de l'impulsion;
  ◦ une amplitude d'onde de pouls droite en calculant une différence entre les amplitudes du premier pic et d'une seconde vallée de l'impulsion;
  ◦ une durée d'onde de pouls en calculant une différence entre un instant de la seconde vallée et un instant de la première vallée de l'impulsion;

◦ un temps de montée en calculant une différence entre un instant du premier pic et un instant de la première vallée de l'impulsion;

◦ un rapport de durée systole-diastole en calculant le rapport entre le temps de montée et une différence entre l'instant de la seconde vallée et l'instant du premier pic de l'impulsion;

- éliminer une impulsion de photopléthysmographie lorsque:

◦ le temps de montée de ladite impulsion est en dehors d'une première plage temporelle; ou
◦ la durée d'onde de pouls de ladite impulsion est en dehors d'une seconde plage temporelle; ou
◦ un rapport entre l'amplitude d'onde de pouls droite et l'amplitude d'onde de pouls gauche est inférieur à une première valeur seuil; ou
◦ le rapport de durée systole-diastole est supérieur à une seconde valeur seuil;

- éliminer une impulsion de photopléthysmographie courante lorsque:

◦ un rapport entre le temps de montée d'une impulsion de photopléthysmographie précédente et le temps de montée de ladite impulsion courante est supérieur à une troisième valeur seuil; ou
◦ un rapport entre la durée d'onde de pouls d'une impulsion de photopléthysmographie précédente et la durée d'onde de pouls de ladite impulsion courante est supérieur à une quatrième valeur seuil; ou
◦ un rapport entre l'amplitude d'onde de pouls d'une impulsion de photopléthysmographie précédente et l'amplitude d'onde de pouls de ladite impulsion courante est supérieur à une cinquième valeur seuil;

- calculer une valeur moyenne des amplitudes des impulsions de photopléthysmographie non éliminées;
- surveiller un nombre prédéterminé de plusieurs impulsions consécutives après la détection d'une impulsion de photopléthysmographie ayant une amplitude inférieure à une fraction prédéterminée de ladite valeur moyenne;
- déclencher une alarme lorsque l'amplitude de ladite pluralité d'impulsions consécutives est inférieure à ladite fraction de la valeur moyenne.

2. Dispositif portable selon la revendication 1,
   **caractérisé en ce que** le dispositif portable est configuré pour filtrer le signal de photopléthysmographie, de préférence en supprimant les fréquences situées en dehors d'une plage de fréquences prédéfinie au moyen d'un filtre passe-bande, et pour utiliser ledit signal de photopléthysmographie filtré dans les étapes restantes de la méthode.

3. Dispositif portable selon la revendication 1 ou 2,
   **caractérisé en ce que** le dispositif portable est configuré pour arrêter l'alarme lorsqu'il détecte, dans une première durée temporelle prédéfinie, une pluralité d'impulsions consécutives ayant des amplitudes supérieures à ladite fraction de la valeur moyenne.

4. Dispositif portable selon l'une quelconque des revendications 1 à 3,
   **caractérisé en ce que** le dispositif portable est configuré pour arrêter l'alarme lorsque:

   - le porteur appuie sur un bouton du dispositif portable afin d'indiquer une fausse alarme; et
   - le porteur appuie sur ledit bouton dans une seconde durée temporelle prédéfinie.

5. Dispositif portable selon l'une quelconque des revendications 1 à 4,
   **caractérisé en ce que** le dispositif portable est configuré pour utiliser des composants GPS du dispositif portable afin de collecter des coordonnées de localisation du porteur et pour partager lesdites coordonnées de localisation avec un tiers.

6. Dispositif portable selon l'une quelconque des revendications 1 à 5,
   **caractérisé en ce que** le dispositif portable est configuré pour exécuter les opérations des revendications 1 à 5 uniquement lorsqu'un résultat d'un test de détection du port est positif.

7. Dispositif portable selon la revendication 6,
   **caractérisé en ce que** le dispositif portable est configuré pour :

- enregistrer une amplitude minimale et une amplitude maximale du signal de photopléthysmographie filtré pendant une troisième durée temporelle après la détection d'une onde de pouls ayant une amplitude supérieure à une sixième valeur seuil;

- calculer une plage de fluctuation en soustrayant l'amplitude minimale de l'amplitude maximale; et

- enregistrer un résultat négatif au test de détection du port lorsque la plage de fluctuation est inférieure à une septième valeur seuil, et enregistrer un résultat positif au test de détection du port lorsque la plage de fluctuation est supérieure à la septième valeur seuil.

8. Dispositif portable selon la revendication 7,
**caractérisé en ce que** le dispositif portable est configuré pour:

- calculer une valeur moyenne des amplitudes minimales d'une pluralité de porteurs; et
- fixer la septième valeur seuil à une fraction de ladite valeur moyenne.

9. Dispositif portable selon l'une quelconque des revendications 6 à 8,
**caractérisé en ce que** le test de détection du port comprend les étapes consistant à:

- mesurer une accélération des mouvements du dispositif portable; et
- enregistrer un résultat négatif au test de détection du port lorsque ladite accélération est inférieure à une huitième valeur seuil pendant une quatrième durée temporelle, et enregistrer un résultat positif au test de détection du port lorsque ladite accélération est supérieure à la huitième valeur seuil pendant la quatrième durée temporelle.

10. Dispositif portable selon la revendication 9,
**caractérisé en ce que** le dispositif portable est configuré pour:

- mesurer l'accélération des mouvements du dispositif portable et collecter une accélération minimale des mouvements du dispositif portable pendant une cinquième durée temporelle;
- calculer une valeur moyenne de l'accélération minimale des mouvements du dispositif portable d'une pluralité de porteurs; et
- fixer la huitième valeur seuil à une fraction de ladite valeur moyenne.

11. Dispositif portable selon la revendication 10,
**caractérisé en ce que** la mesure de l'accélération des mouvements du dispositif portable comprend les étapes consistant à:

- collecter des données d'accélération selon un axe x, un axe y et un axe z à partir d'un accéléromètre intégré dans le dispositif portable;
- calculer une dérivée temporelle des données d'accélération selon l'axe x, une dérivée temporelle des données d'accélération selon l'axe y et une dérivée temporelle des données d'accélération selon l'axe z;
- calculer l'accélération du mouvement du dispositif portable en calculant la racine carrée de la somme des carrés de chacune des dérivées temporelles des données d'accélération.

12. Dispositif portable selon l'une quelconque des revendications 1 à 11,
**caractérisé en ce que** le dispositif portable est configuré pour enregistrer un événement de chute lorsque l'accélération du mouvement du dispositif portable est supérieure à une neuvième valeur seuil dans une sixième durée temporelle.

13. Dispositif portable selon la revendication 12,
**caractérisé en ce que** le dispositif portable est configuré pour:

- collecter un horodatage du pic d'accélération lors de l'enregistrement de la chute du porteur dans la sixième durée temporelle;
- collecter un horodatage du début d'impact lorsque l'accélération du mouvement du dispositif portable est inférieure à une dixième valeur seuil dans la sixième durée temporelle;
- collecter un horodatage de la fin d'impact lorsque l'accélération du mouvement du dispositif portable est supérieure à une onzième valeur seuil dans la sixième durée temporelle; et
- calculer des paramètres d'accélération proportionnels à l'accélération du mouvement du dispositif portable à chaque horodatage ou à une combinaison arithmétique d'au moins deux horodatages.

14. Dispositif portable selon l'une quelconque des revendications 12 ou 13,
    **caractérisé en ce que** le dispositif portable est configuré pour entraîner un réseau neuronal destiné à détecter un arrêt cardiaque au moyen d'un modèle de gradient boosting et d'un ensemble de données d'apprentissage comprenant les paramètres d'accélération calculés et les événements de chute enregistrés, en minimisant une fonction de perte sur l'ensemble de données d'apprentissage.

15. Dispositif portable selon l'une quelconque des revendications précédentes, ledit dispositif portable comprenant un ordinateur chargé d'un programme informatique,
    **caractérisé en ce que** ledit programme est agencé pour amener l'ordinateur à exécuter les opérations selon l'une quelconque des revendications précédentes.

PPG front end

Do not measure raw PPG ← No — Is the user wearing the bracelet according to the wearing detection?

Yes

Raw PPG

Filter the raw PPG using a bandpass filter between 0.67 Hz and 5 Hz.

Determining amplitude threshold. Is the PPG amplitude below the threshold of 33% of the average peak amplitude?

Yes

Three axis accelerometer signal measuring levels of acceleration

Is there still a PPG signal present based on the Signal Quality Indicator? ← No — Is the PPG signal amplitude above the threshold for 4 consecutive pulses?

Yes

Fall detection algorithm

No

Are the normal PPG pulses coming back within 5 seconds?

Yes

Stop alarm

Has a fall been detected by the fall detection algorithm in the 10 seconds before the Audible alarm?

No

Yes/No

Audible Alarm ← No — Are 4 consecutive normal pulses detected?

Yes

A fall in the 10 seconds before the alarm increases the probability of a cardiac arrest.

Does the user determine it is a false alarm and press the button within 10 seconds to stop alarm?

No

Internal GPS component is turned on and detects location of the user in units of latitude and longetude

**FIG. 1**

**EP 4 512 321 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200305737 A